# EUROPEAN PATENT APPLICATION

(11) **EP 2 184 055 A1**
(43) Date of publication of application: **12.05.2010**
(21) Application number: 08168594.3
(22) Date of filing: 07.11.2008
(51) Int. Cl.: A61K 9/16, A61K 9/20, A61K 9/50, A61P 3/10, A61K 31/4439, A61K 31/64, A61K 45/06

(54) **Process for preparing solid dosage forms of rosiglitazone maleate**

(71) Applicant: LEK Pharmaceuticals d.d., 1526 Ljubljana (SI)
(72) Inventor: Stanic Ljubin, Tijana, 1526 Ljubljana (SI); Reven, Sabastjan, 1526 Ljubljana (SI); Jaklic, Miha Tomaz, 1526 Ljubljana (SI); Svete, Peter, 1526 Ljubljana (SI)

(57) **Abstract**

The invention relates to a process for preparing a solid pharmaceutical composition rosiglitazone maleate, comprising the step of adsorption of rosiglitazone maleate onto carrier particles (C) during a dry mixing process.

## Description

The present invention relates to a process for preparing a solid pharmaceutical composition comprising rosiglitazone maleate and optionally, additional active ingredients. The active ingredient can be administered in low doses and with excellent uniformity. The adsorption of rosiglitazone maleate onto the surface of specific pharmaceutical excipients was found to significantly improve the content uniformity in final dosage forms and reduces the likelihood of segregation during processing in the presence of excipients with different particle size.

Rosiglitazone is a pharmaceutical product described e.g. in EP-A 0 306 228 having the name 5-[4-[2-(N-methyl-N-(2-pyridyl)amino)ethoxy]-benzyl]-2,4-thiazolidinedione. The maleate salt of rosiglitazone is described e.g. in EP-A 0 658 161 and has the following formula:

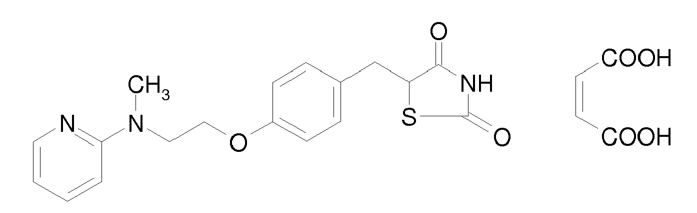

Rosiglitazone belongs to the thiazolidinedione class of compounds and is known for years as a very potent antidiabetic compound. More particularly rosiglitazone maleate is one preferred drug for non-insulin dependent diabetes mellitus (NIDDM). Diabetes mellitus is a complex, chronically progressive disease, which affects the function of the kidneys, eyes, vascular and nervous systems.

Rosiglitazone was marketed some years ago as a stand-alone drug and in combination with other anti-diabetic drugs, for example in combination with metformin or with glimepiride. Different crystalline salts of rosiglitazone and particularly of rosiglitazone maleate are commercially used due to improved stability and solubility in water in comparison to the rosiglitazone base.

The compound rosiglitazone exists in different polymorphic forms. The characteristics like chemical stability and solubility are influenced by the conformation and orientation of molecules in the unit cell defining a particular polymorphic form of a substance. Therefore changes in the polymorphic form of a pharmaceutical compound during manufacturing process or during life cycle of the product can lead to changed performance characteristics of the pharmaceutical product.

There are several polymorphic forms of rosiglitazone and rosiglitazone maleate described in the literature. The documents WO 99/31093, WO 99/31094 and WO 99/31095 are directed to hydrates of rosiglitazone maleate and their use in pharmaceutical compositions for the treatment of diabetes mellitus. WO 02/26737 relates to novel polymorphic or pseudopolymorphic forms of rosiglitazone maleate and pharmaceutical compositions with a higher activity as antibiabetic agents. This document discloses polymorphic forms I, II, II and IV, which are obtained from different solvents like ethanol, acetone, methanol and 1,4-dioxane. A special preparation method of solid pharmaceutical compositions, for example of tablets is not described in WO 02/26737.

Furthermore, WO 00/64892 and WO 00/64896 deal with polymorphic forms of rosiglitazone maleate which are characterized by spectroscopic data and can be obtained by crystallisation from ethanol solution. The according pharmaceutical compositions are prepared by conventional methods.

Important challenges in the manufacturing of solid dosage forms of very low-dose drugs are to ensure homogeneous distribution of the drug substance in the tablet blend and the homogeneity in the tablets produced. One technical problem is how to adequately distribute the drug substance evenly among the large amount of excipient particles. Particulate solids, once mixed, have the tendency to segregate by virtue of differences in the shape, size, density and other variables of the particles of which they are composed. This process occurs during mixing, as well as during subsequent handling of the completed mix. If a drug substance composed of particles, without further processing, is used in tablet manufacture, the tablets so produced lack content uniformity and do not possess an acceptable drug content. Poor content uniformity has been shown in the literature to cause a decrease in bioavailability and can also cause toxicity, e.g. if the amount of drug substance is too high. The pharmaceutical industry employs different methods for the preparation of granulations that subsequently are converted to finished dosage forms.

Among these methods for the preparation are wet granulation, dry granulation and direct compression. The simplest way of manufacturing tablets is to blend all the ingredients as dry powders and to tablet them (direct compression). This is a process rarely successful for low dose drugs. A common problem with this process is segregation of the powder blend during tabletting.

A preferred known method for formulating low dose drugs is known as wet granulation. In a wet granulation process, a drug is blended with excipients and then a granulation liquid is added, so that a wet granulate is formed. Granulation liquid can be water or another solvent or the solution of binder in water or another solvent. Wet granulation can be also carried out with granulation liguid comprising dispersed drug, which prevents loss of a low dose drugs.Wet granulate is dried in a separate step. This method is effective but has some disadvantages. The drying step requires special equipment, and generally involves high temperatures which may degrade e. g. labile drugs. The presence of water can initiate the polymorphic change of a drug. Also, the use of the binder normally requires the further inclusion of a disintegrant component to help the tablet, which is cohesive, to disperse in the stomach.

Fluid bed wet granulation has also been used to achieve content uniformity of low dose tablets. In this process, the micronised drug is blended as a powder with other excipients. Then the mixture is loaded into a fluid bed granulator, and the powders are agglomerated by spraying on a solution of a binder. The process of drying can take place concomitantly. The process incorporates a separate blending step prior to granulation, a specialized equipment and precise optimization of the process parameters.

In US 4,489,026, a process for preparing a solid dosage form is disclosed, wherein a fine spray of a dilute solution of a pharmaceutically active agent is sprayed onto excipient particles. This process involves very slowly spraying and includes the use of a volatile inert solvent preferably an organic solvent having a boiling point lower than 80°C. The process is carried out in an open coating pan. A continuous flow of air then dries the product during the spraying process. This process was applied to drugs with a unit dose of several µg or less. The spray rate is however limited to 1-ml/min, which makes the process suitable only for very small batch-sizes. The example quoted in US 4,489,026 prepared 1000 tablets. The weight ratio of solution to carrier used in US 4,489,026 was 15%. Furthermore, the use of volatile organic liquids today is regarded as a significant hazard, requiring solvent-recovery processes and explosion-proof equipment.

US 4,898,736 describes a simplified version of a spraying process, suitable for unit doses of 50-1000 µg. In the disclosed process, the active ingredient is dissolved in an easily evaporated solvent such as ethanol, methanol, acetone or tetrahydrofuran and is simply blended with excipients in a ratio of 2.26% or 6.75%. The blend is then dried, followed by lubrication and tabletting. This process is in principle suitable for commercial scale manufacture, but the described process causes problems associated with the use of volatile organic solvents.

One of the objects of the present invention is to provide an improved process for preparing solid pharmaceutical compositions, wherein there is no need of solvents and the resulting solid compositions shows improved drug content uniformity.

Surprisingly it was found that a solid pharmaceutical composition comprising rosiglitazone maleate and optionally another active ingredient can be prepared by adsorption of the particles of rosiglitazone maleate onto specific pharmaceutical carrier particles during a dry-mixing process. The inventive process shows inter alia the following advantages:
- There is no need for a drying step as in wet granulation. This simplifies processing and reduces production costs. Furthermore, heat labile drugs do not suffer at the temperature required for drying.
- Because the use of volatile organic solvents is avoided, no problem may arise in relation to environmental protection, safety of workers, and residual solvents.
- For drugs which are highly potent, the omission of the drying step makes it easier to contain dust improving safety for the factory worker.
- According to the new process, the active pharmaceutical ingredient is not in contact with water, so no change in polymorphism can be initiated, which can lead to changes of performance characteristics of the pharmaceutical product.

One object of the present is a process for preparing a solid pharmaceutical composition comprising rosiglitazone maleate comprising the step of adsorption of rosiglitazone maleate onto carrier particles (C) during a dry mixing process.

The adsorption step can be performed by manual blending the active ingredient with the carrier particles or in mixing equipment. In a preferred embodiment of the invention the adsorption step is carried out with a high shear mixer.

According to the present invention, a process for preparing a solid pharmaceutical composition is provided which comprises admixing specific carrier particles (C) with active ingredient rosiglitazone maleate. During the process of mixing carrier particles and the active ingredient, the particles of active ingredient are evenly adhered onto the surface of carrier particles.

Another object of the present invention is a process for preparing a solid pharmaceutical composition comprising rosiglitazone maleate comprising the steps of:
a) preparing a dry mixture (M1) comprising carrier particles (C), rosiglitazone maleate as active ingredient,
   wherein the particles of rosiglitazone maleate are adhered onto the surface of carrier particles (C), and optionally other components (A)
b) converting the resulting dry mixture (M1) into solid pharmaceutical composition.

The carrier particles (C) can in principle be any suitable, directly compressible pharmaceutically acceptable excipient. Preferably said carrier particles (C) are selected from particles consisting of lactose, lactose monohydrate, spray-dried lactose, microcrystalline cellulose, silicified microcrystalline cellulose, or spray dried material composed of lactose and pulverized cellulose, such as Cellactose^{®} 80 or spray dried material composed of lactose and microcrystalline cellulose, such as MicroceLac^{®},. More preferably, said carrier particles (C) are selected from particles consisting of spray-dried lactose and microcrystalline cellulose.

The ratio of the amounts of carrier particles (C) and active ingredient rosiglitazone maleate in dry mixture (M1) can be from 70:30% w/w to 99,5:0,5% w/w. The preferable ratio of the amounts of carrier particles (C) and active ingredient rosiglitazone maleate in dry mixture (M1) is from 70:30% w/w to 95:5% w/w. The more preferable ratio of the amounts of carrier particles (C) and active ingredient rosiglitazone maleate in dry mixture (M1) is about 90:10% w/w.

Optional additives (other components A) in the dry mixture (M1) include conventionally used additives in pharmaceuticals, particular in preparing tablets. Such other components can be e. g. anti-adherents, binders, disintegrants, fillers/diluents, glidants, lubricants, flavours, colors preservatives, sorbents and/or sweeteners.
The amount of other components (A) can be up to 50%.w/w, preferably up to 30% w/w, more preferably up to 10% w/w.

In a first step a) the active ingredient rosiglitazone maleate is adhered onto the surface of carrier particles during a dry-mixing process. The first step a) includes blending and mixing of active ingredient rosiglitazone maleate and the carrier particles (C) and optionally other components (A) in one or more steps, particularly with a high shear mixer, with bin blender or manually. If the first step a) is carried manually or in bin blender, the mixture is sieved between the different mixing steps. If the first step a) is carried in a high shear mixer, the mixture is optionally sieved between different mixing steps. In a preferred embodiment of the invention, the active ingredient rosiglitazone maleate and the carrier particles (C) are mixed in a high shear mixer. The time of mixing procedure is in the range of 1 to 30 minutes, preferably 10 to 30 minutes, more preferably 20 to 30 minutes.

Preferably, the sieving step of the process is carried out through a screen with meshes in the range of 0.2 to 1 mm, more preferred through a sieve with meshes in the range of 0.25 to 0.5 mm. The sieving step respectively the sieving steps help to produce a homogenous distribution of active ingredient rosiglitazone maleate without agglomerates in the dry mixtures for tableting. But it is not obligatory to carry out a sieving step in the present inventive process if the mixing is carried out in high shear mixer.

The solid pharmaceutical composition of the present invention can be any form of solid pharmaceutical composition known in the field of the pharmaceutical technology. Preferably, the solid pharmaceutical composition is in the form of a tablets, pills, capsules, caplets, lozenges, sachets or powder. Tablets may be suitably coated (film coated tablets, pills). Capsule formulations may cover both soft and hard capsules. A pharmaceutical compositions according to the present invention is more preferably in form of tablets.
The step b) may be performed by the processes which are conventional in pharmaceutical technology.
Preferably, the step b) includes the compressing of the resulting dry mixture (M1) into tablets. This can be particularly carried out in a rotary tabletting machine. The film coating may be applied onto the rapidly disintegrating tablet cores of the present invention by the processes which are conventional in pharmaceutical technology. Preferably, the film coating is applied by spraying the film coating dispersion. The film coating dispersion is prepared by dispersing the dry components of film coating in water.

In a preferred embodiment of the present invention, the process for preparing a solid pharmaceutical composition comprising rosiglitazone maleate comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
b) converting the resulting dry mixture (M1) into solid pharmaceutical composition, preferably by compressing of the resulting dry mixture (M1) into tablets.

In another preferred embodiment of the present invention, the process for preparing a solid pharmaceutical composition comprising rosiglitazone maleate comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
b) converting the resulting dry mixture (M1) into solid pharmaceutical composition, preferably by compressing of the resulting dry mixture (M1) into tablets

In another preferred embodiment of the present invention, the process for preparing a solid pharmaceutical composition comprising rosiglitazone maleate comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
b) converting the resulting dry mixture (M1) into solid pharmaceutical composition, preferably by compressing of the resulting dry mixture (M1) into tablets

In a more preferred embodiment of the present invention, the process for preparing a solid pharmaceutical composition comprising rosiglitazone maleate comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
      wherein the active ingredient rosiglitazone maleate and the carrier particles (C) are mixed in manually or in bin blender, and the mixture is sieved between the different mixing steps,
b) converting the resulting dry mixture (M1) into solid pharmaceutical composition, preferably by compressing of the resulting dry mixture (M1) into tablets.

In another more preferred embodiment of the present invention, the process for preparing a solid pharmaceutical composition comprising rosiglitazone maleate comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
      wherein the active ingredient rosiglitazone maleate and the carrier particles (C) are mixed in manually or in bin blender, and the mixture is sieved between the different mixing steps,
b) converting the resulting dry mixture (M1) into solid pharmaceutical composition, preferably by compressing of the resulting dry mixture (M1) into tablets

In another more preferred embodiment of the present invention, the process for preparing a solid pharmaceutical composition comprising rosiglitazone maleate comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
      wherein the active ingredient rosiglitazone maleate and the carrier particles (C) are mixed in manually or in bin blender, and the mixture is sieved between the different mixing steps,
b) converting the resulting dry mixture (M1) into solid pharmaceutical composition, preferably by compressing of the resulting dry mixture (M1) into tablets.

In a more preferred embodiment of the present invention, the process for preparing a solid pharmaceutical composition comprising rosiglitazone maleate comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
      wherein the active ingredient rosiglitazone maleate and the carrier particles (C) are mixed in a high shear mixer, and optionally the mixture is sieved between the different mixing steps,
b) converting the resulting dry mixture (M1) into solid pharmaceutical composition, preferably by compressing of the resulting dry mixture (M1) into tablets.

In another more preferred embodiment of the present invention, the process for preparing a solid pharmaceutical composition comprising rosiglitazone maleate comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
      wherein the active ingredient rosiglitazone maleate and the carrier particles (C) are mixed in a high shear mixer, and optionally the mixture is sieved between the different mixing steps,
b) converting the resulting dry mixture (M1) into solid pharmaceutical composition, preferably by compressing of the resulting dry mixture (M1) into tablets

In another more preferred embodiment of the present invention, the process for preparing a solid pharmaceutical composition comprising rosiglitazone maleate comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
      wherein the active ingredient rosiglitazone maleate and the carrier particles (C) are mixed in a high shear mixer, and optionally the mixture is sieved between the different mixing steps,
b) converting the resulting dry mixture (M1) into solid pharmaceutical composition, preferably by compressing of the resulting dry mixture (M1) into tablets.

Another object of the present invention is a solid pharmaceutical composition comprising rosiglitazone maleate and carrier particles (C) obtained by the process of the present invention.

Another object of the present invention is a solid pharmaceutical composition comprising rosiglitazone maleate and carrier particles (C), wherein the particles of rosiglitazone maleate are adhered onto the surface of carrier particles (C), obtained by the process of the present invention.

Optionally, solid dosage forms comprising rosiglitazone maleate prepared according to the process of the present invention comprise one or more additional active ingredients, preferably selected from known antidiabetic compounds, for example pioglitazone, metformin, glimepirid, glibenclamid The phase comprising additional active ingredient used in the process of the present invention can be wet granulate, dry granulate, complex, solid dispersion or physical mixture of additional active ingredient or it can be pure additional active ingredient itself.

Another object of the present invention is a process for preparing a solid pharmaceutical composition comprising rosiglitazone maleate and second active ingredient comprising the step of adsorption of rosiglitazone maleate onto carrier particles (C) during a dry mixing process.

Another object of the present invention is a process for preparing a solid pharmaceutical composition comprising rosiglitazone maleate and second active ingredient comprising the steps of:
a) preparing a dry mixture (M1),
   comprising carrier particles (C), rosiglitazone maleate as active ingredient, wherein the particles of rosiglitazone maleate are adhered onto the surface of carrier particles (C), and optionally other components (A)
b) preparing a final mixture (M2)
   comprising the following components: dry mixture (M1), a phase comprising second active ingredient (Z), disintegrant (D) and other components (B),
c) converting the resulting final mixture (M2) into solid pharmaceutical composition.

The carrier particles (C) can in principle be any suitable, directly compressible pharmaceutically acceptable excipient. Preferably said carrier particles (C) are selected from particles consisting of lactose, lactose monohydrate, spray-dried lactose, microcrystalline cellulose, silicified microcrystalline cellulose, or spray dried material composed of lactose and pulverized cellulose, such as Cellactose^{®} 80 or spray dried material composed of lactose and microcrystalline cellulose, such as MicroceLac^{®},. More preferably, said carrier particles (C) are selected from particles consisting of spray dried lactose and microcrystalline cellulose.

The ratio of the amounts of carrier particles (C) and active ingredient rosiglitazone maleate in dry mixture (M1) can be from 70:30% w/w to 99,5:0,5% w/w. The preferable ratio of the amounts of carrier particles (C) and active ingredient rosiglitazone maleate in dry mixture (M1) is from 70:30% w/w to 95:5% w/w. The more preferable ratio of the amounts of carrier particles (C) and active ingredient rosiglitazone maleate in dry mixture (M1) is about 90:10% w/w.

Optional additives (other components A) in the dry mixture (M1) include conventionally used additives in pharmaceuticals, particular in preparing tablets. Such other components can be e. g. anti-adherents, binders, disintegrants, fillers/diluents, glidants, lubricants, flavours, colors preservatives, sorbents and/or sweeteners.
The amount of other components (A) can be up to 50%.w/w, preferably up to 30% w/w, more preferably up to 10% w/w.

The second active ingredient (Z) is selected preferably from known antidiabetic compounds, for example pioglitazone, metformin, glimepirid, glibenclamid In a preferred embodiment of the invention the second active ingredient (Z) is glimepiride. The phase comprising second active ingredient (Z) that enters final mixture (M2) can be wet granulate, dry granulate, complex, solid dispersion or physical mixture of second active (Z) or it can be pure second active (Z) itself. In particular the phase comprising second active ingredient (Z) is a granulate comprising second active ingredient (Z), prepared by wet granulation process from the active, fillers, binder and disintegrants.

The disintegrant (D) used according to the present invention in preparing final mixture M2 can be selected e. g. from disintegrant known in the art for preparing solid pharmaceutical dosage forms. Typical examples of disintegrants used in direct powder compression are: sodium starch glycolate, crospovidone, croscarmelose sodium, starch, low-substituted hydroxypropyl cellulose. The preferred disintegrant (D) is sodium starch glycolate.

Preferably the other component (B) in the final mixture (M2) is selected from a usable lubricant (L), particularly magnesium stearate, calcium stearate, stearic acid and/or glyceryl behenate.and optionally glidant.

In a first step a) the active ingredient rosiglitazone maleate is adhered onto the surface of carrier particles during a dry-mixing process. The first step a) includes blending and mixing of active ingredient rosiglitazone maleate and the carrier particles (C) and optionally other components (A) in one or more steps, particularly with a high shear mixer, with bin blender or manually. If the first step a) is carried manually or in bin blender, the mixture is sieved between the different mixing steps. If the first step a) is carried in a high shear mixer, the mixture is optionally sieved between different mixing steps. In a preferred embodiment of the invention, the active ingredient rosiglitazone maleate and the carrier particles (C) are mixed in a high shear mixer. The time of mixing procedure is in the range of 1 to 30 minutes, preferably 10 to 30 minutes, more preferably 20 to 30 minutes.

Preferably, the sieving step of the process is carried out through a screen with meshes in the range of 0.2 to 1 mm, more preferred through a sieve with meshes in the range of 0.25 to 0.5 mm. The sieving step respectively the sieving steps help to produce a homogenous distribution of active ingredient rosiglitazone maleate without agglomerates in the dry mixtures for tabletting. But it is not obligatory to carry out a sieving step in the present inventive process if the mixing is carried out in high shear mixer..

Preferably, the step b) includes blending the dry mixture (M1) with a phase comprising second active ingredient (Z) ,disintegrant (D) and other components (B), determined as above More preferably, the step b) includes blending the dry mixture (M1) with granulate of second active ingredient Z and optionally a disintegrant and other components (B) determined as above. In a preferred embodiment, the blend is lubricated with magnesium stearate.

The solid pharmaceutical composition of the present invention can be any form of solid pharmaceutical composition known in the field of the pharmaceutical technology. Preferably, the solid pharmaceutical composition is in the form of a tablets, pills, capsules, caplets, lozenges, sachets or powder. Tablets may be suitably coated (film coated tablets, pills). A pharmaceutical compositions according to the present invention is more preferably in form of tablets.
The step b) may be performed by the processes which are conventional in pharmaceutical technology.
Preferably, the step c) includes the compressing of the resulting final mixture (M2) into tablets. This can be particularly carried out in a rotary tabletting machine. The film coating may be applied onto the rapidly disintegrating tablet cores of the present invention by the processes which are conventional in pharmaceutical technology. Preferably, the film coating is applied by spraying the film coating dispersion. The film coating dispersion is prepared by dispersing the dry components of film coating in water.

In a further embodiment of the invention, other thiazolidinedione derivates or their pharmaceutical acceptable salts are used as active ingredient in mixture M1, for example one or several drug compounds from the group comprising of rosiglitazone, troglitazone, ciglitazone, pioglitazone or their pharmaceutical acceptable salts.

The invention furthermore relates to a solid pharmaceutical composition comprising rosiglitazone maleate and carrier particles (C), which composition is prepared as described above. In a preferred embodiment, the pharmaceutical composition is an immediate release tablet comprising two active ingredients rosiglitazone maleate and a second active ingredient (Z), *e.g*. glimepiride. Preferably the solid dosage form has a target potency of 4/1, 4/2 and 4/4 mg (rosiglitazone/glimepiride) in about 200 mg tablet (2 % loading of rosiglitazone maleate).

In another preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
b) preparing a final mixture (M2)
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
b) preparing a final mixture (M2)
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing the resulting final mixture (M2) into tablets

In another more preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
      wherein the active ingredient rosiglitazone maleate and the carrier particles (C) are mixed in manually or in bin blender, and the mixture is sieved between the different mixing steps,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another preferred embodiment of the present invention, the process for preparing a solid pharmaceutical composition comprising rosiglitazone maleate and glimepiride comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in manually or in bin blender, and the mixture is sieved between the different mixing steps,
b) preparing a final mixture (M2) comprising:
   - 20-50% w/w of dry mixture (M1),
   - 50-80% w/w of a granulate glimepiride,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
   wherein the active ingredient rosiglitazone maleate and the carrier particles (C) are mixed in a high shear mixer,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
   wherein the active ingredient rosiglitazone maleate and the carrier particles (C) are mixed in a high shear mixer,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in a high shear mixer,
b) preparing a final mixture (M2) comprising:
   - 10-90% w/w of dry mixture (M1),
   - 10-90% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-50% w/w of other components (A),
   wherein the active ingredient rosiglitazone maleate and the carrier particles (C) are mixed in a high shear mixer,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing the resulting final mixture (M2) into tablets.

In another more preferred embodiment of the present invention, the process for preparing a solid pharmaceutical composition comprising rosiglitazone maleate and second active ingredient comprises the steps of:
a) preparing a dry mixture (M1) comprising
   - 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-30% w/w of other components (A),
   wherein the active ingredient rosiglitazone maleate and the carrier particles (C) are mixed in a high shear mixer,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets

In another preferred embodiment of the present invention, the process for preparing a solid pharmaceutical composition comprising rosiglitazone maleate and second active ingredient comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in a high shear mixer,
b) preparing a final mixture (M2) comprising:
   - 20-50% w/w of dry mixture (M1),
   - 50-80% w/w of a phase comprising second active ingredient (Z), preferably a granulate,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another preferred embodiment of the present invention, the process for preparing a solid pharmaceutical composition comprising rosiglitazone maleate and glimepiride comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in a high shear mixer,
b) preparing a final mixture (M2) comprising:
   - 20-50% w/w of dry mixture (M1),
   - 50-80% w/w of a granulate comprising glimepiride,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

In another preferred embodiment of the present invention, the process for preparing a solid pharmaceutical composition comprising rosiglitazone maleate and glimepiride comprises the steps of:
a) preparing a dry mixture (M1) comprising
   - 90-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
   - 0-10% w/w of other components (A),
   wherein the active ingredient rosiglitazone maleate and the carrier particles (C) are mixed in a high shear mixer and carrier particles (C) are selected from particles consisting of spray dried lactose and microcrystalline cellulose.,
b) preparing a final mixture (M2) comprising:
   - 20-50% w/w of dry mixture (M1),
   - 50-80% w/w of a granulate comprising glimepiride,
   - 0.5-10% w/w of disintegrant (D) and
   - 0-5% w/w other components (B),
c) converting the resulting final mixture (M2) into solid pharmaceutical composition, preferably by compressing of the resulting final mixture (M2) into tablets.

Another object of the present invention is a solid pharmaceutical composition comprising rosiglitazone maleate, carrier particles (C) and second active ingredient obtained according to the process of the present invention.

Another object of the present invention is a solid pharmaceutical composition comprising rosiglitazone maleate and carrier particles (C), wherein the particles of rosiglitazone maleate are adhered onto the surface of carrier particles (C), and second active ingredient, obtained by the process of the present invention.

For comparison to the inventive process for preparing a solid pharmaceutical composition with a low dose drug, the solid pharmaceutical composition was prepared by different conventional tablet forming processes wherein the active ingredient rosiglitazone maleate was not formulated and processed according to the present invention. Instead of being incorporated into dry mixture (M1), rosiglitazone maleate was included into solid pharmaceutical composition as follows:
a) rosiglitazone maleate by itself without formulating (see example 2),
b) dry granulate of rosiglitazone maleate was used instead of dry mixture (M1) (see example 3),
c) wet granulate (with ethanol) was used instead of dry mixture (M1) (see example 4).

However none of the mentioned technologies gave comparable results to the inventive process, meaning proper content uniformity or the absence of the segregation during technology procedure. The inventive process for preparing solid pharmaceutical compositions shows minimized segregation potential and proper content uniformity.

The present invention is further directed to the use of a solid pharmaceutical composition described above for the treatment and/or prophylaxis of diabetes mellitus, conditions associated with diabetes mellitus and certain complications thereof.
The present invention is further directed to the solid pharmaceutical composition described above for the treatment and/or prophylaxis of diabetes mellitus, conditions associated with diabetes mellitus and certain complications thereof. The present invention is further directed to the use of a solid pharmaceutical composition described above for the manufacture of a medicament for the treatment and/or prophylaxis of diabetes mellitus, conditions associated with diabetes mellitus and certain complications thereof.
Diabetes mellitus is a complex, chronically progressive disease, which affects e. g. the function of the kidneys, eyes, vascular and nervous systems.
Preferably the solid pharmaceutical composition is used in the treatment and/or prophylaxis of diabetes mellitus, preferably Type II diabetes mellitus, more preferably the non-insulin dependent diabetes mellitus (NIDDM). Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein include renal diseases, especially renal disease associated with the development of Type II diabetes, including diabetic nephropathy, glomerulonephritis, glomerular sclerosis, nephritic syndrome, hypertensive nephrosclerosis and end stage renal disease.

The content of the pharmaceutically active compound per unit of solid unit dosage form is determined by a common Content Uniformity Test. The uniformity of content is a pharmaceutical analysis technique applied especially for the quality control of solid pharmaceutical compositions. A sample of about 10 dosage units is sampled at random. Using a suitable analytical method (like HPLC), the individual content of active ingredient is assayed.

Requirements of the (USP) Content Uniformity Test are determined as follows:
The requirement are met if the content of not more than one individual content is outside the limit of 85 to 115 % of the average content and none is outside the limit of 75 % to 125 % of the average content. The content uniformity of the active ingredient can also be determined by the relative standard Devitation (RSD). The rosiglitazone maleate assay was analyzed using HPLC (High performance liquid chromatography). The single tablets of a sample of about 10 tablets are dissolved in water and the content of RM was analysed by HPLC method.

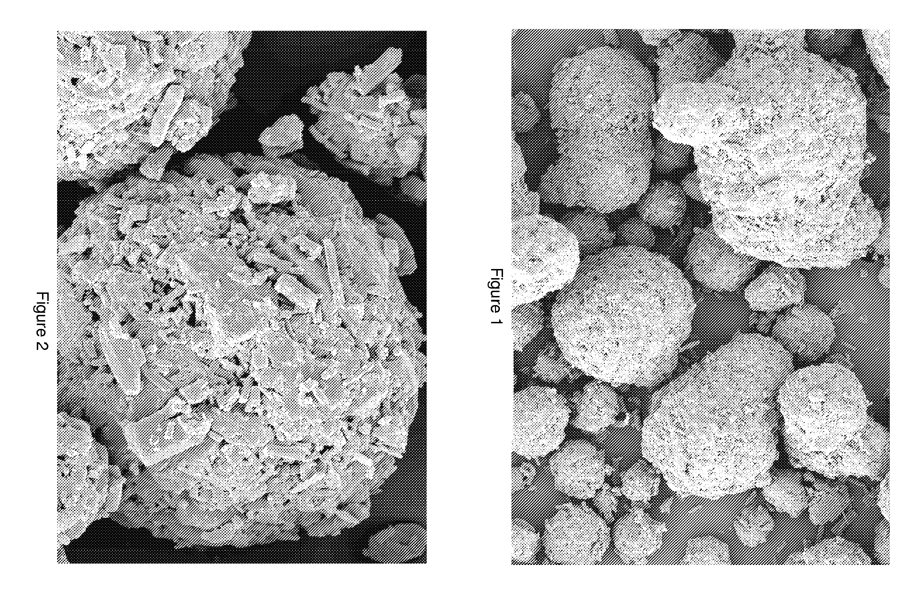

SEM pictures of the mixtures, where active ingredient rosiglitazone maleate is adsorbed onto the surface of the carrier particles (C) are shown in the enclosed Figures. 1 and 2.

Figures 1 and 2 show spray dried lactose, where active ingredient is adhered onto the surface.

The following examples illustrate the invention and describe the technological procedures.

### Part I - Preparation of tablets including the adsorption of rosiglitazone maleate onto the surface of carrier particles (C)

### Example 1A:

Composition of dry mixture M1 comprising rosiglitazone maleate:

| | mg/tbl. |
|---|---|
| Rosiglitazone maleate | 5.30 |
| Spray dried lactose | 54.70 |
| Total | 60.00 |

Rosiglitazone maleate was blended with spray dried lactose (mean particle size 125 µm) as described in following text.
Rosiglitazone maleate was mixed with 20 % of the total amount of spray dried lactose and sieved through the sieve 0.3 mm. The resulting mixture was mixed with 40% of the total amount of spray dried lactose and sieved through the sieve 0.3 mm.
The remaining spray dried lactose (40% of the total amount) was sieved through the same sieve 0.3 mm, that was used for the sieving of above described mixtures, in order to pick up the residuals of rosiglitazone maleate.

The sieved spray dried lactose was added to the last mixture of RM and lactose and blended with it in bin blender resulting in rosiglitazone dry mixture M1.

Composition of final mixture M2 for tabletting:

| | mg/tbl. |
|---|---|
| Granulate of glimepiride | 140.00 |
| Dry mixture M1 | 60.00 |
| Sodium starch glycolate | 5,00 |
| Magnesium stearate | 1.00 |
| Total | 206.00 |

The dry mixture M1 prepared as described above was blended with granulate of glimepiride and sodium starch glycolate, the blend was lubricated with magnesium stearate and compressed into tablets on rotary tabletting machine. Tablets were sampled in the beginning, middle and the end of tabletting and the rosiglitazone maleate assay was analyzed. Also the content uniformity on the average sample was determined.

Assay determination:
1. Transfer 10 tablets to a 200 ml volumetric flask
2. Dilute with solvent* to volume and stir with a magnetic stirrer until the tablets have disintegrated. Then place the flask for 20 minutes in an ultrasonic bath and stir for additional 15 minutes with a magnetic stirrer. Mix well. Termostate suspension to room temperature. Pass through a filter, discarding the first 2 ml of the filtrate.

Uniformity of dosage units - uniformity of content determination
1. transfer 1 tablet to a 20 mL volumetric flask
2. dilute with solvent* to volume and stir with a magnetic stirrer until the tablets have disintegrated. Then place the flask for 20 minutes in an ultrasonic bath and stir for additional 15 minutes with a magnetic stirrer. Mix well. Termostate suspension to room temperature and then pass through a filter, discarding the first 2 ml of the filtrate.
* solvent = water : acetonitrile = 200 : 800 (V/V)

The HPLC analysis were performed with a Waters system equipped with a XBridge C18, 3,5 µm, 150 X 4.6 mm column which was maintained in a column oven at 30 °C. The mobile phase A consisted of a 72 : 28 (V/V) mixture of phosphate buffer, pH = 2,5 / and acetonitrile and mobile phase B consisted of a 30 : 70 (V/V) mixture of phosphate buffer, pH = 2,5 / and acetonitrile. The flow rate was 1.5 mL/min, using following gradient

| Time (minutes) | %A |
|---|---|
| 0 | 100 |
| 4 | 0 |
| 7 | 0 |
| 7,5 | 100 |
| 12 | 100 |

and the detection wavelength was 230 nm.

Results of the assay of rosiglitazone maleate are presented in Table 1. Table 1 shows average content of rosiglitazone and the relative standard deviation (RSD), which is related to the content uniformity.

### Example 1B

Composition of dry mixture M1 comprising rosiglitazone maleate:

| | mg/tbl. |
|---|---|
| Rosiglitazone maleate | 5.30 |
| Spray dried lactose | 58.70 |
| Total | 64.00 |

Rosiglitazone maleate was blended with spray dried lactose (mean particle size 125 µm) in high shear mixer as described in following text. The mixture of rosiglitazone maleate and spray dried lactose was mixed for 10 minutes in high shear mixer with impeller and sieved through the sieve 0.3 mm. Then the mixture was additionally mixed for 10 minutes in high shear mixer with impeller and chopper and sieved through trough the sieve 0.3 mm. After sieving, mixture was mixed for additional 2 minutes in high shear mixer with impeller and chopper resulting in dry mixture M1.

Composition of final mixture M2::for tabletting:

| | mg/tbl. |
|---|---|
| Granulate of glimepiride | 133.30 |
| Dry mixture M1 | 64.00 |
| Sodium starch glycolate | 8.00 |
| Magnesium stearate | 1.00 |
| Total | 206.30 |

The dry mixture M1 prepared as described above was blended with granulate of glimepiride and sodium starch glycollate. The blend was lubricated with magnesium stearate and compressed into tablets on rotary tabletting machine. A sample of tablets was sampled and analyzed as described in example 1A. Results of assay of rosiglitazone maleate are shown in Table 1.

### Example 1C

Composition of dry mixture M1 comprising rosiglitazone maleate::

| | mg/tbl. |
|---|---|
| Rosiglitazone maleate | 5.30 |
| Cellulose, microcrystalline | 58.70 |
| Total | 64.00 |

Rosiglitazone maleate was blended with microcrystalline cellulose (nominal particle size 180 µm) in high shear mixe ras described in following text.
The mixture of rosiglitazone maleate and microcrystalline cellulose was mixed for 10 minutes in high shear mixer with impeller and chopper and sieved through the sieve 0.5 mm. Then the mixture was additionally mixed for 2 minutes in high shear mixer with impeller and chopper and for additional 10 minutes with impeller resulting in rosiglitazone maleate dry mixture M1.

Composition of final mixture M2 for tabletting:

| | mg/tbl. |
|---|---|
| Granulate of glimepiride | 139.00 |
| Dry mixture M1 | 64.00 |
| Sodium starch glycolate | 5.00 |
| Magnesium stearate | 1.00 |
| Total | 209.00 |

The dry mixture M1 prepared as described above was blended with granulate of glimepiride and sodium starch glycollate. The blend was lubricated with magnesium stearate and compressed into tablets on rotary tabletting machine.

A sample of tablets was sampled and analyzed as described in example 1 A. Results of assay of rosiglitazone maleate are shown in Table 1.

**Table 1**

| ASSAY | EXAMPLE 1A | | EXAMPLE 1B | | EXAMPLE 1C | |
|---|---|---|---|---|---|---|
| Beginning of tabletting | 97.2 % | RSD n.a. | 99.5 % | RSD 0,50 | 99.0 % | RSD 0,11 |
| Middle of tabletting | 96.9 % | RSD n.a. | 100.0 % | RSD 0,76 | 101.6 % | RSD 1,03 |
| End of tabletting | 96.3 % | RSD n.a. | 99.0 % | RSD 0,32 | 99.7 % | RSD 0,87 |
| Content uniformity on average sample | 97.2 % | RSD 0,96 | 98.6 % | RSD 0,57 | 99.0 % | RSD 1,65 |

Comparing the results in Table 1 with the results of conventional methods like direct compression (example 2, Table 2), dry granulation (example 3, Table 3) or wet granulation (example 4, Table 4) shows that the process according to the invention leads to dosage forms with minimized segregation potential and with proper content uniformity (higher rosiglitazone maleate assay in the tablet and low RSD means high uniformity).

### Example 1D

Composition of dry mixture M1 comprising rosiglitazone maleate:

| | mg/tbl. |
|---|---|
| Rosiglitazone maleate | 5.30 |
| Cellulose, microcrystalline | 91.70 |
| Sodium starch glycolate | 2.50 |
| Magnesium stearate | 0.50 |
| Total | 100.00 |

Rosiglitazone maleate was blended with microcrystalline cellulose (nominal particle size 180 µm) in high shear mixer as described in following text.
The mixture of rosiglitazone maleate and microcrystalline cellulose was mixed for 10 minutes in high shear mixer with impeller and chopper. Then the mixture was additionally mixed for 2 minutes in high shear mixer with impeller and chopper and for additional 10 minutes with impeller. Sodium starch glycolate was added to the mixture in high shear mixer and the mixture was mixed for additional 2 minutes with impeller. Then magnesium stearate is added to the mixture in high shear mixer and the mixture was mixed for additional 1 minute with impeller resulting in rosiglitazone maleate dry mixture M1. Mixture M1 was then compressed into tablets on rotary tabletting machine.

### Example 1E

Composition of the granulate of glimepiride:

| | |
|---|---|
| Glimepiride | 4,000 |
| Lactose monohydrate | 80,000 |
| Sodium starch glycolate | 10,000 |
| Hypromellose | 5,000 |
| Cellulose, microcrystalline | 40,000 |
| Demineralized water | 56,000 |
| Total | 139,000 |

Glimepiride, lactose monohydrate, microcrystalline cellulose, hypromellose and sodium starch glycolate were mixed for 3 minutes in high shear mixer with impeller and chopper. Than the demineralized water was added to the mixture while mixed with impeller for 3 minutes. The wetted mass was granulated in high shear mixer for 2 minutes. Obtained wet granulate was transferred to fluid bed drier and dried. Dried granulate was screened through 0,5 mm sieve by screening mill.

### Part II - Comparative methods of preparing tablets

### Example 2 - Dry addition of rosiglitazone maleate

Composition of final mixture for tabletting:

| | mg/tbl. |
|---|---|
| Granulate of glimepiride | 140.00 |
| Rosiglitazone maleate | 5.30 |
| Magnesium stearate | 0.88 |
| Total | 146.18 |

Rosiglitazone maleate was blended with the granulate of glimepiride as described in following text.

Rosiglitazone maleate was mixed with 10% of the total amount granulate of glimepiride and sieved trough the sieve 1.0 mm.
The resulting mixture was mixed with 20% of the total amount granulate of glimepiride and sieved trough the sieve 1.00 mm.
The remaining granulate of glimepiride (70% of the total amount) was sieved through the same sieve 1.0 mm, that was used for the sieving the other mixtures, in order to pick up the residuals of rosiglitazone maleate, and added to the first mixture described above and blended with it in bin blender.
The resulting mixture was lubricated with magnesium stearate and compressed into tablets on rotary tabletting machine.
A sample of tablets was sampled and analyzed as described in example 1 A. Results of assay of rosiglitazone maleate are shown in Table 2.

**Table 2**

| ASSAY | % | RSD (%) |
|---|---|---|
| Beginning of tabletting | 95.1 | 3.74 |
| Middle of tabletting | 95.6 | 2.35 |
| End of tabletting | 98.2 | 1.22 |
| Content uniformity on average sample | 90.1 | 9.16 |

### Example 3 - Dry granulate of rosiglitazone maleate

Composition of rosiglitazone maleate dry granulate:

| | mg/tbl. |
|---|---|
| Rosiglitazone maleate | 5.30 |
| Lactose | 53.20 |
| Sodium starch glycolate | 1.50 |
| Magnesium stearate | 0.25 |
| | 60.25 |

Rosiglitazone maleate was blended with in bin blender.
The resulting mixture was blended with sodium starch glycollate in bin blender, the blend was lubricated with magnesium stearate and compressed into tablets on rotary tabletting machine. The tablets of rosiglitazone maleate are crushed by screening by oscillating bar mill resulting rosiglitazone dry granulate.

Composition of final mixture for tabletting:

| | mg/tbl. |
|---|---|
| Granulate of glimepiride | 140,00 |
| Dry granulate of rosiglitazone maleate | 60,00 |
| Sodium starch glycolate | 5,00 |
| Magnesium stearate | 1,00 |
| Total | 206,00 |

Rosiglitazone maleate dry granulate prepared as described above was blended with sodium starch glycollate and the granulate of glimepiride, the blend was lubricated with magnesium stearate and compressed into tablets on rotary tabletting machine.

A sample of tablets was sampled and analyzed as described in example 1 A. Results of assay of rosiglitazone maleate are shown in Table 3.

**Table 3**

| ASSAY | % | RSD (%) |
|---|---|---|
| Beginning of tabletting | 97.9 | **n.a**. |
| Middle of tabletting | 98.1 | **n.a.** |
| End of tabletting | 94.3 | **n.a.** |
| Content uniformity on average sample | 97.2 | 3.39 |

### Example 4 - Wet granulate of rosiglitazone maleate

Composition of rosiglitazone maleate granulate:

| | mg/tbl. |
|---|---|
| Rosiglitazone maleate | 5.30 |
| Lactose, monohydrate | 43.70 |
| Sodium starch glycolate | 1.50 |
| Povidone K 25 | 1.50 |
| Cellulose microcrystalline | 8.00 |
| Ethanol | 20.00 |
| Total granulate | 60.00 |

Rosiglitazone maleate, lactose monohydrate, povidone K 25, microcrystalline cellulose, and sodium starch glycolate were mixed for 3 minutes in high shear mixer with impeller and chopper. Than the ethanol was added to the mixture while mixed with impeller and chopper for 3 minutes. The wetted mass was granulated in high shear mixer for 2 minutes. Obtained wet granulate was transferred to fluid bed drier and dried. Dried granulate was screened through 0,5 mm sieve by screening mill.

Composition of final mixture for tabletting:

| | mg/tbl. |
|---|---|
| Granulate of active ingredient Z | 140.00 |
| RM wet granulat | 60.00 |
| Sodium starch glycolate | 5.00 |
| Magnesium stearate | 1,00 |
| | 206,00 |

Rosiglitazone maleate wet granulate prepared as described above was blended with the granulate of glimepiride and sodium starch glycollate, the blend was lubricated with magnesium stearate and compressed into tablets on rotary tabletting machine.

A sample of tablets was sampled and analyzed as described in example 1 A. Results of assay of rosiglitazone maleate are shown in Table 4.

**Table 4**

| ASSAY | % | RSD (%) |
|---|---|---|
| Beginning of tabletting | 97.4 | **n.a.** |
| Middle of tableting | 98.6 | **n.a.** |
| End of tabletting | 97.7 | **n.a.** |
| Content uniformity on average sample | 101.8 | 3.09 |

## Claims

1. A process for preparing a solid pharmaceutical composition comprising rosiglitazone maleate comprising the step of adsorption of rosiglitazone maleate onto carrier particles (C) during a dry mixing process.

2. Process according to claim 1 comprising the steps of:
a) preparing a dry mixture (M1)
comprising carrier particles (C), rosiglitazone maleate as active ingredient,
wherein the particles of rosiglitazone maleate are adhered onto the surface of carrier particles (C), and optionally other components (A)
b) converting the resulting dry mixture (M1) into solid pharmaceutical composition.

3. Process according to claim 1 or claim 2 comprising the steps of:
a) preparing a dry mixture (M1) comprising
- 50-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w,
- 0-50% w/w of other components (A),
b) converting the resulting dry mixture (M1) into solid pharmaceutical composition.

4. Process according to any one of claim 1 to 3, wherein said solid pharmaceutical composition further comprises the second active ingredient (Z).

5. Process according to the claim 4 comprising the steps of:
a) preparing a dry mixture (M1),
comprising carrier particles (C), rosiglitazone maleate as active ingredient, wherein the particles of rosiglitazone maleate are adhered onto the surface of carrier particles (C), and optionally other components (A)
b) preparing a final mixture (M2)
comprising the following components: dry mixture (M1), a phase comprising second active ingredient (Z), disintegrant (D) and other components (B),
c) converting the resulting dry mixture (M1) into solid pharmaceutical composition.

6. Process according to the claim 4 or the claim 5 comprising the steps of:
a) preparing a dry mixture (M1) comprising
- 70-100% w/w of carrier particles (C) and rosiglitazone maleate as active ingredient, wherein the particles of rosiglitazone maleate are adhered onto the surface of carrier particles (C) and wherein the ratio of the amounts of carrier particles (C) and active substance rosiglitazone is from 70:30% w/w to 99,5:0,5% w/w, preferably from 70:30% w/w to 95:5% w/w, more preferably about 90:10% w/w
- 0-30% w/w of other components (A),
b) preparing a final mixture (M2) comprising:
- 10-90% w/w of dry mixture (M1),
- 10-90% w/w of a phase comprising second active ingredient (Z), preferably a granulate,
- 0.5-10% w/w of disintegrant (D) and
- 0-5% w/w other components (B),
c) converting the resulting dry mixture (M1) into solid pharmaceutical composition.

7. Process according to one of the claims 1 or 6, wherein said carrier particles (C) are selected from particles consisting of lactose, lactose monohydrate, spray-dried lactose, microcrystalline cellulose, silicified microcrystalline cellulose, spray dried material composed of lactose and pulverized cellulose, and spray dried material composed of lactose and microcrystalline cellulose.

8. Process according to one of the claims 2 to 7, wherein said step a) of preparing dry mixture (m1) is carried out with a high shear mixer, with bin blender or manually and a mixture is optionally sieved between different mixing steps.

9. A solid pharmaceutical composition comprising rosiglitazone maleate, carrier particles (C) and optioanally second active ingredient (Z) obtained by the process to any one of the claims 1 to 8.

10. The use of a solid pharmaceutical composition according to the claim 9 for the treatment and/or prophylaxis of diabetes mellitus, conditions associated with diabetes mellitus and certain complications thereof.
